# EUROPEAN PATENT SPECIFICATION

(11) **EP 1 344 543 B1**
(45) Date of publication and mention of the grant of the patent: **07.06.2006**
(21) Application number: 03004815.1
(22) Date of filing: 04.03.2003
(51) Int. Cl.: A61M 1/36

(54) **Venous blood reservoir in an extracorporeal circuit**
Venöse Blutkammer in einem extrakorporalen Kreislauf
Réservoir de sang veineux dans un circuit extracorporel

(30) Priority: 12.03.2002 IT MI20020526
(43) Date of publication of application: 17.09.2003
(73) Proprietor: SORIN GROUP ITALIA S.R.L., 20121 Milano (IT)
(72) Inventor: Ghelli, Nicola, 40018 S. Pietro in Casale, Bologna (IT); Panzani, Ivo, 41037 Mirandola, (Prov. of Modena) (IT); Tommasi, Gabriele, 41032 Cavezzo, (Prov. of Modena) (IT)
(74) Representative: Modiano, Micaela Nadia

(56) References cited:
- EP-A- 0 820 775
- EP-A- 1 053 760
- EP-A- 1 070 509
- US-A- 4 490 331
- US-B1- 6 337 049

## Description

The present invention relates to a venous blood reservoir in extracorporeal circuit.

It is known that many surgical procedures entail the need to divert the blood of the patient into an extracorporeal circuit that comprises blood accumulation reservoirs. One of the reservoirs is the so-called venous reservoir, which receives the blood, known as venous blood, from the patient before it is passed through an oxygenation apparatus, which is also included in the extracorporeal circuit. Another of the reservoirs is designed to contain blood collected in the operating field, which once appropriately filtered can be returned to the patient; this reservoir is known as a cardiotomy reservoir.

The venous reservoirs of the prior art receive the blood that arrives from the patient by gravity, and this entails a forced placement of such reservoirs at a lower level than the operating field, which is often poorly compatible with the limited space available. It should also be noted that the blood circulation provided by gravity is not always as efficient as would be desirable. One way to increase the efficiency of drainage is to apply a vacuum to the interior of the reservoir. However, such vacuum assisted reservoirs have not, in the past, been combined with a cardiotomy reservoir to form a reservoir system having features desired by the user.

EP-A-0 820 775 discloses a combined blood reservoir having a combination of features as set forth in the precharacterizing portion of the appended claim 1, which comprises a lower venous blood reservoir separated from an upper cardiotomy reservoir by a partition, an axially movable column extending through the partition, and ducts formed in the partition, all in selected communication with both the venous and cardiotomy reservoirs. An air outlet connector is provided at the top of the cardiotomy reservoir.

US-6 337 049 discloses a venous reservoir with vacuum augmented venous drainage provided by a gas port connected to a vacuum source.

US-A4 490 331 discloses a unitary extracorporeal blood processing system which incorporates an upper reservoir container having a blood gas separator, a cardiotomy reservoir and a venous reservoir. A vacuum is drawn upon an upper portion of the reservoir container which carries the blood gas separator.

EP-A-1 070 509 discloses an air-tight venous reservoir having a vacuum source connected to the top of the reservoir.

EP-A-1 053 760 discloses a cardiotomy reservoir having a bottom which forms the cover of a venous reservoir, and a blood conduction channel expending between an outlet at a bottom of the cardiotomy reservoir and an inlet at the bottom of the venous reservoir. A vacuum port is provided at the top of the cardiotomy reservoir.

The aim of the present invention is therefore to provide a venous reservoir that ensures intensive drainage of the blood of the patient without any limitation in selecting its location. Within this aim, an object of the invention is to devise a venous reservoir combined with a cardiotomy reservoir.

In accordance with the invention, there is provided a venous blood reservoir as defined in the appended claims.

Further characteristics and advantages will become better apparent from the description of a preferred but not exclusive embodiment of the invention, illustrated only by way of non-limitative example in the accompanying drawings, wherein:
Figure 1 is a general sectional view of the device of this invention, taken along the line I-I of Figure 2;
Figure 2 is a sectional view, taken along the line II-II of Figure 1;
Figure 3 is an enlarged-scale view of the upper region of Figure 2 in a different functional condition; and
Figure 4 is a detail view of the device of this invention.

With reference to the figures, reference number 1 designates the enclosure of the combined device. Enclosure 1 includes a first chamber or portion comprising a venous reservoir 2 and a second chamber or portion comprising a cardiotomy reservoir 4. Venous reservoir 2 is positioned below cardiotomy reservoir 4 and the two reservoirs are separated by a generally horizontal partition 3. It should be understood, however, that the concepts of the present invention disclosed herein are not limited to a venous reservoir combined with a cardiotomy reservoir but are equally applicable to stand-alone venous reservoirs.

The venous reservoir 2 is provided with an inlet connector 5 for the inflow of the venous blood, into a central region 5a that is defined by a wall 6. Wall 6 comprises a bubble rupturing substance and is surrounded by a filter 6a. Venous reservoir 2 is also provided with an outlet connector 7 for the outflow of the blood. Thus, blood entering venous reservoir 2 through inlet connector 5 is directed to central region 5a. Before the blood exits the venous reservoir, it passes through wall 6 and filter 6a where bubbles and other undesirable materials are removed.

The cardiotomy reservoir 4 is provided with an inlet connector 8 for the inflow of the blood that arrives from the operating field. Inlet connector 8 opens into a region 8a that is defined by wall 9, which comprises a bubble rupturing substance. Wall 9 is surrounded by a filter 9a.

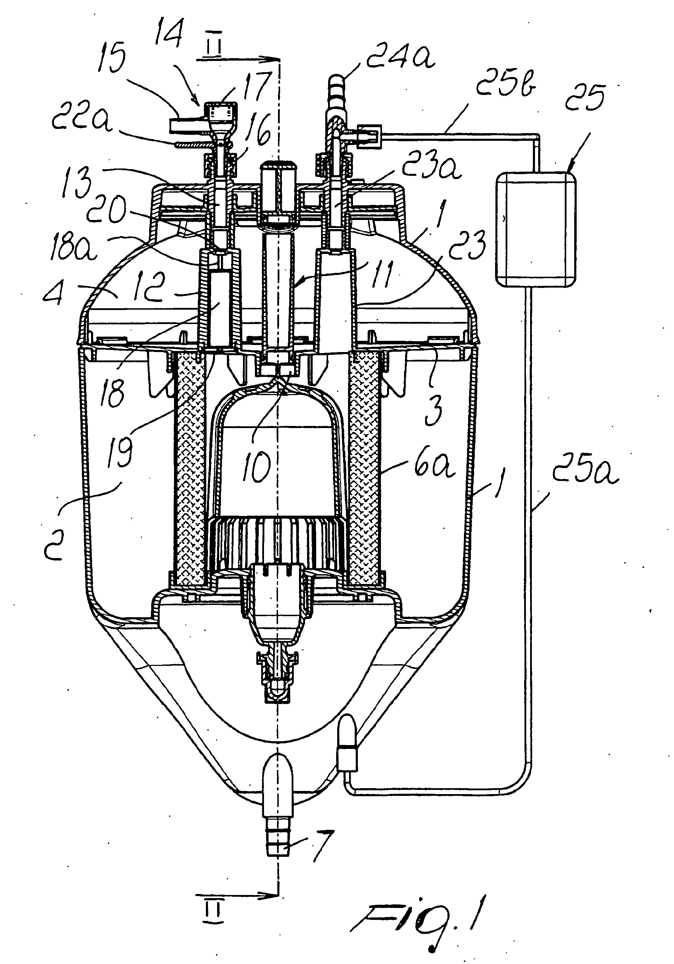

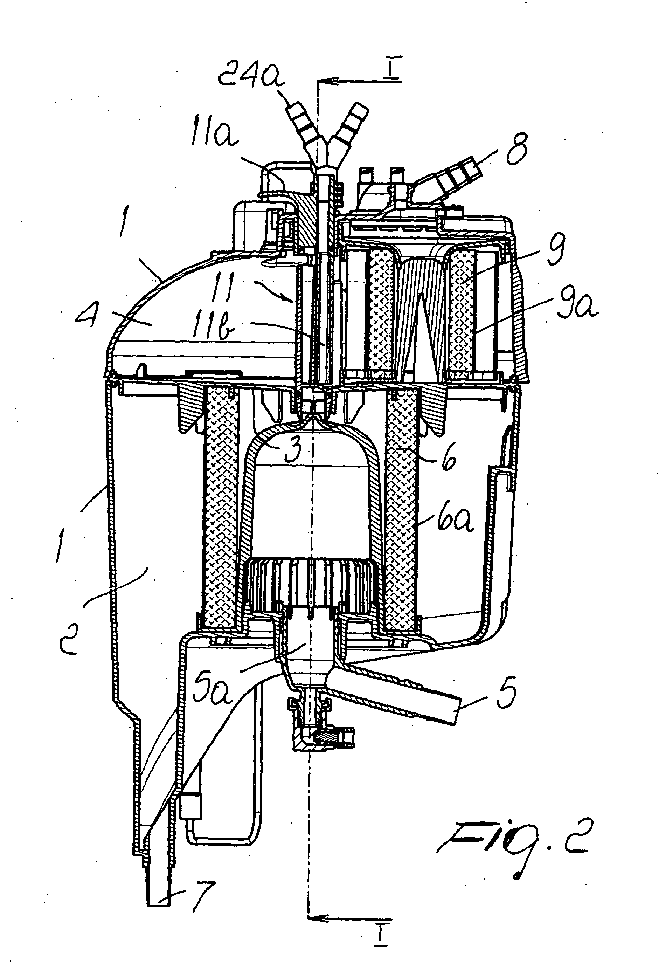

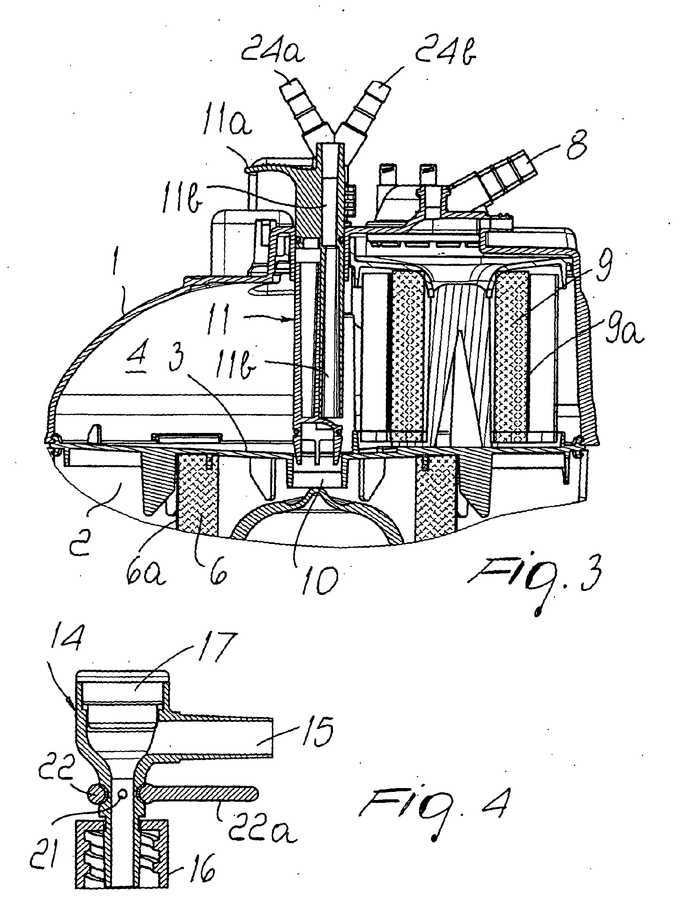

The partition 3 has a central opening 10, which connects the cardiotomy reservoir 4 to the venous reservoir 2. Means are provided for closing central opening 10. Manual action on tab 11a will cause column 11 to slide between a lower stroke limit position (as shown in Figures 1 and 2), thus closing opening 10, and an upper stroke limit position (as shown in Figure 3), in which opening 10 is open in order to allow emptying of the blood contained in the cardiotomy reservoir into the underlying venous reservoir.

If it is necessary to empty the cardiotomy reservoir while opening 10 is closed, the duct 11b provided in the column 11 is used. Duct 11b is open at the lower end into the cardiotomy reservoir to allow aspiration through the open upper end.

An important feature of the invention is that partition 3, comprising the upper lid of the venous reservoir 2, is provided with a connector 12, which comprises a duct which communicates with a lower end of duct 13 formed within the upper structure of the enclosure 1. As best seen in Figure 4, an upper end of duct 13 joins coupling 14 which has a vacuum port comprising a connector 15 that is adapted to be connected to a line that is attached to a vacuum source.

The connection of the coupling 14 to the upper end of the duct 13 is provided by means of the threaded ring 16, which allows rotation of the coupling. A safety valve 17 contained within coupling 14 prevents vacuum from exceeding a desired level.

During use of the device, vacuum is applied to venous reservoir 2 by connecting connector 15 to a vacuum source. The formation of vacuum in the venous reservoir 2 results in an effective drainage of the blood that arrives from the patient, and this occurs regardless of the level at which the venous reservoir is arranged with respect to the operating field.

As best seen in Figure 1, within the connector 12 there is a float 18, which in the inactive or open position (shown in Figure 1) rests on a frame 19. Float 18 is provided with needle 18a, which is adapted to close an opening 20 that connects the connector 12 to the duct 13 when float 18 is moved to the active or open position. If the blood contained in the venous reservoir 2 reaches a level where it enters connector 12 creating a situation that might lead to invasion of the vacuum line on the part of the blood, float 18 will be caused by the blood to move upwardly in turn moving needle 18a to a position which blocks opening 20 to duct 13, thus preventing blood from entering the vacuum line.

The return of the float 18 to the inactive position, once the emergency has ended, is facilitated by the temporary connection of the space within duct 13 that lies above the float to atmospheric pressure. This is accomplished by opening a small hole 21 provided in the coupling 14 by deformation of an elastic ring 22, which normally keeps the hole closed, the deformation being caused by the operator by manually acting on a tab 22a.

A turret 23 opens into venous reservoir 2 and extends from partition 3. It is joined by means of duct 23a to connectors 24a and 24b for connection to lines that are normally closed in order to maintain a desired amount of vacuum in the reservoir 2. The turret is adapted for the introduction, when necessary, of medical liquids into the reservoir.

The venous reservoir according to the invention is provided with a control system comprising a device capable of automatic adjustment of the level of the blood contained therein based upon pressure within the reservoir. The control system includes a computer schematically designated by the reference numeral 25. The pressure at the base of the reservoir and the negative pressure at the top of the reservoir are continuously sensed and provided to the computer. The pressure at the top and bottom of the reservoir is sensed through ducts 25a and 25b, respectively. The computer adjusts the amount or degree of vacuum that is present in the reservoir based on the sensed pressure data to maintain the proportion between the flow-rate of the incoming blood and the flow-rate of the outgoing blood, to keep the level of the blood in the venous reservoir substantially constant.

The described invention is susceptible of numerous modifications and variations, all of which are within the scope of the appended claims; thus, for example, the device for automatic adjustment of the level of the blood in the reservoir can be provided in any manner and the cardiotomy reservoir can be omitted.

Furthermore, connector 15 adapted to be connected to the vacuum line can branch out from the wall of the enclosure 1 proximate to the partition 3.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A vacuum assisted venous blood reservoir (2) in extracorporeal circuit, comprising a blood inlet connector (5) connected to a line for drawing blood from the patient, a blood outlet connector (7) for the outflow of the blood toward an oxygenation apparatus, and a cardiotomy reservoir (4) that is connected monolithically to said reservoir (2) in a position that lies above the upper lid (3) of said reservoir (2), said lid (3) being provided with an opening (10) for flow between said reservoir (2) and said cardiotomy reservoir (4) that is provided with closure means (11,11a,11b) adapted to be managed by the assigned operator, **characterized in that** it comprises, at a partition comprising the upper lid (3) of the venous reservoir (2), a connector (12) comprising a duct extending through the cardiotomy reservoir (4) and communicating with a lower end of a duct (13) formed within the upper structure of the enclosure of the cardiotomy reservoir (4) and adapted for connection to a line that reaches a vacuum source for applying vacuum to the venous reservoir (2).

2. The reservoir according to claim 1, **characterized in that** it comprises a coupling (14) adapted to be arranged at the connector (12) at the upper lid (3) of said reservoir (2), and is provided with a connector (13) for connection to the line that reaches a vacuum source, and comprising a safety valve (17) against unwanted degrees of vacuum.

3. The reservoir according to one or more of the preceding claims, **characterized in that** it comprises a float (18) adapted to exclude the connection of the reservoir (2) to the line that reaches a vacuum source if the level of the blood in said reservoir (2) rises excessively, so as to prevent any possibility of contamination of said line.

4. The reservoir according to claim 3, **characterized in that** the float (18) is accommodated within said connector (12), so as to rest in an inactive condition on a frame (19) arranged at the base of the connector (12), and is provided with a needle (18a) adapted to close an opening (20) provided proximate to the top of said connector (12),

5. The reservoir according to one or more of claims 2-4, **characterized in that** the coupling (14) adapted to be arranged at the connector (12) provided at the upper lid (3) of said reservoir (2) comprises a hole (21) for outside connection, which is normally closed by way of plug means (22) adapted to be removed easily.

6. The reservoir according to claim 5, **characterized in that** the means for plugging the outside connection hole of the coupling comprise a ring (22) made of elastic material which is provided with a grip tab (22a) and is adapted to be associated with the outer surface of the coupling (14) at the hole (21).

7. The reservoir according to one or more of the preceding claims, **characterized in that** it comprises a device (25) for automatic adjustment of the level of the blood contained in said reservoir (2).

8. The reservoir according to claim 7, **characterized in that** the device for automatic adjustment of the level of the blood contained in the reservoir comprises adjustment means (25) adapted to act on the degree of vacuum inside said reservoir (2).

9. The reservoir according to claim 7, **characterized in that** the device for automatic adjustment of the level of the blood contained in the reservoir comprises adjustment means (25) that are adapted to act on the degree of vacuum inside said reservoirs (2) on the basis of a continuous sensing of the pressure at the base of the reservoir (2) and of said degree of vacuum.

10. The reservoir according to claim 1, **characterized in that** the closure means for closing the opening for flow between the reservoir (2) and the cardiotomy reservoir (4) comprise a column (11) that can slide upon manual actuation and is provided with a duct (11b) that is open at both ends, respectively, onto the inside of said cardiotomy reservoir (4) and toward the outside.

## Patentansprüche

1. Vakuum-unterstützter Behälter (2) für venöses Blut in extrakorporalem Kreislauf, aufweisend: einen Bluteinlassanschluss (5), der mit einer Leitung verbunden ist, die vom Patienten Blut abzapft, einen Blutauslassanschluss (7), durch den das Blut zu einer Sauerstoffanreicherungsvorrichtung herausfließt, und einen Kardiotomie-Behälter (4), der monolithisch mit dem Behälter (2) in einer oberhalb des oberen Deckels (3) des Behälters (2) befindlichen Position verbunden ist, wobei der Deckel (3) mit einer Öffnung (10) für einen Durchfluss zwischen dem Behälter (2) und dem Kardiotomie-Behälter (4) versehen ist, die mit einer Verschlusseinrichtung (11, 11a, 11b) versehen ist, welche ausgebildet ist, um durch die verantwortliche Bedienperson bedient zu werden, **dadurch gekennzeichnet, dass** er, an einer Trennwand, die den oberen Deckel (3) des Behälters (2) für venöses Blut beinhaltet, ein Verbindungsstück (12) aufweist, das einen Kanal beinhaltet, der sich durch den Kardiotomie-Behälter (4) hindurch erstreckt und mit einem unteren Ende eines Kanals (13) in Verbindung steht, der innerhalb der oberen Struktur der Umhüllung des Kardiotomie-Behälters (4) ausgebildet ist und für eine Verbindung mit einer Leitung ausgebildet ist, die zu einer Vakuumquelle führt, um ein Vakuum an den für venöses Blut dienenden Behälter (2) anzulegen.

2. Behälter nach Anspruch 1, **dadurch gekennzeichnet, dass** er ein Kopplungsstück (14) aufweist, das ausgebildet ist, um am Verbindungsstück (12) am oberen Deckel (3) des Behälters (2) angeordnet zu werden, und mit einem Anschluss (15) versehen ist, der eine Verbindung zu der zu einer Vakuumquelle führenden Leitung herstellt und ein Sicherheitsventil (17) aufweist, das einen unerwünschten Grad des Vakuums verhindern soll.

3. Behälter nach einem oder mehreren der vorhergehenden Ansprüche, **dadurch gekennzeichnet, dass** er einen Schwimmer (18) aufweist, der ausgebildet ist, um die Verbindung des Behälters (2) zu der zu einer Vakuumquelle führenden Leitung zu schließen, wenn der Pegel des in dem Behälter (2) befindlichen Blutes übermäßig ansteigt, um so jegliche mögliche Verunreinigung der Leitung zu verhindern.

4. Behälter nach Anspruch 3, **dadurch gekennzeichnet, dass** der Schwimmer (18) in dem Verbindungsstück (12) untergebracht ist, so dass er in einem inaktiven Zustand auf einem auf der Basis des Verbindungsstücks (12) angeordneten Rahmen (19) aufliegt, und mit einer Nadel (18a) versehen ist, die ausgebildet ist, um eine Öffnung (20) zu verschließen, die in der Nähe der Oberseite des Verbindungsstücks (12) vorgesehen ist.

5. Behälter nach einem oder mehreren der Ansprüche 2 bis 4, **dadurch gekennzeichnet, dass** das Kopplungsstück (14), das ausgebildet ist, um an dem am oberen Deckel (3) des Behälters (2) vorgesehenen Verbindungsstück (12) angeordnet zu werden, ein Loch (21) für eine Verbindung nach Außen aufweist, das normalerweise mittels einer Stopfeneinrichtung (22) verschlossen ist, die ausgebildet ist, um leicht entfernt zu werden.

6. Behälter nach Anspruch 5, **dadurch gekennzeichnet, dass** die Einrichtung zum Zustöpseln des Verbindungslochs nach Außen des Kopplungsstückes einen Ring (22) aus einem elastischen Material aufweist, der mit einer Grifflasche (22a) versehen ist und ausgebildet ist, um in Zusammenhang mit der am Loch (21) befindlichen Außenfläche des Kopplungsstücks (14) zu stehen.

7. Behälter nach einem oder mehreren der vorhergehenden Ansprüche, **dadurch gekennzeichnet, dass** er eine Vorrichtung (25) für eine automatische Einstellung des Pegels des in dem Behälter (2) enthaltenen Blutes aufweist.

8. Behälter nach Anspruch 7, **dadurch gekennzeichnet, dass** die Vorrichtung zur automatischen Einstellung des Pegels des im Behälter enthaltenen Blutes Einstelleinrichtungen (25) aufweist, die ausgebildet sind, um auf den Grad des Vakuums im Inneren des Behälters (2) einzuwirken.

9. Behälter nach Anspruch 7, **dadurch gekennzeichnet, dass** die Vorrichtung zur automatischen Einstellung des Pegels des im Behälter enthaltenen Blutes Einstelleinrichtungen (25) aufweist, die ausgebildet sind, um auf den Vakuumgrad im Inneren des Behälters (2) einzuwirken, und zwar auf Basis eines kontinuierlichen Messen des Druckes an der Basis des Behälters (2) und des Vakuumgrades.

10. Behälter nach Anspruch 1, **dadurch gekennzeichnet, dass** die Verschlusseinrichtung zum Schließen der Durchflussöffnung zwischen dem Behälter (2) und dem Kardiotomie-Behälter (4) einen Pfeiler (11) aufweist, der durch manuelle Betätigung gleitend verschoben werden kann und mit einem Kanal (11b) versehen ist, welcher an beiden Enden offen ist, und zwar zum Inneren des Kardiotomie-Behälters (4) hin und nach Außen hin.

## Revendications

1. Réservoir de sang veineux (2) assisté sous vide dans un circuit extracorporel, comprenant un raccord d'entrée de sang (5) relié à une ligne d'aspiration du sang à partir du patient, un raccord de sortie de sang (7) pour l'écoulement du sang vers un appareil d'oxygénation, et un réservoir de cardiotomie (4) qui est relié de façon monolithique audit réservoir (2) dans une position qui se trouve au-dessus du couvercle supérieur (3) dudit réservoir (2), ledit couvercle (3) étant muni d'une ouverture (10) pour l'écoulement entre ledit réservoir (2) et ledit réservoir de cardiotomie (4) qui est équipé de moyens de fermeture (11, 11 a, 11 b) conçus pour être gérés par l'opérateur désigné,
**caractérisé en ce qu'**il comprend, au niveau d'une séparation comprenant le couvercle supérieur (3) du réservoir veineux (2), un raccord (12) comprenant un conduit traversant le réservoir de cardiotomie (4) et communiquant avec une extrémité inférieure d'un conduit (13) réalisé dans la structure supérieure de l'enceinte du réservoir de cardiotomie (4) et conçu pour relier une ligne qui arrive à une source de vide pour appliquer un vide au réservoir veineux (2).

2. Réservoir selon la revendication 1,
**caractérisé en ce qu'**il comprend un accouplement (14) conçu pour être agencé au niveau du raccord (12), au niveau du couvercle supérieur (3) dudit réservoir (2), et est muni d'un raccord (15) pour la liaison à la ligne qui arrive à une source de vide, et comprenant une valve de sécurité (17) contre des degrés de vide non voulus.

3. Réservoir selon une ou plusieurs des revendications précédentes,
**caractérisé en ce qu'**il comprend un flotteur (18) conçu pour empêcher la liaison du réservoir (2) à la ligne qui arrive à une source de vide si le niveau de sang dans ledit réservoir (2) s'élève de façon excessive, de façon à empêcher toute possibilité de contamination de ladite ligne.

4. Réservoir selon la revendication 3,
**caractérisé en ce que** le flotteur (18) est logé dans ledit raccord (12), de façon à reposer dans un état inactif sur un siège (19) agencé au niveau de la base du raccord (12), et est muni d'une aiguille (18a) conçue pour fermer une ouverture (20) prévue à proximité du sommet dudit raccord (12).

5. Réservoir selon une ou plusieurs des revendications 2-4,
**caractérisé en ce que** l'accouplement (14) conçu pour être agencé au niveau du raccord (12) prévu au niveau de l'extrémité supérieure (3) dudit réservoir (2), comprend un trou (21) pour la liaison vers l'extérieur, qui est normalement fermé par l'intermédiaire, de moyens d'obturation (22) conçus pour être retirés facilement.

6. Réservoir selon la revendication 5,
**caractérisé en ce que** les moyens pour obturer le trou de liaison vers l'extérieur de l'accouplement comprennent un anneau (22) réalisé en une matière élastique, qui est prévu avec une patte de préhension (22a) et est conçu pour être associé à la surface externe de l'accouplement (14) au niveau du trou (21).

7. Réservoir selon une ou plusieurs des revendications précédentes,
**caractérisé en ce qu'**il comprend un dispositif (25) pour le réglage automatique du niveau du sang contenu dans ledit réservoir (2).

8. Réservoir selon la revendication 7,
**caractérisé en ce que** le dispositif pour le réglage automatique du niveau du sang contenu dans le réservoir comprend des moyens de réglage (25) conçus pour agir sur le degré de vide à l'intérieur dudit réservoir (2).

9. Réservoir selon la revendication 7,
**caractérisé en ce que** le dispositif pour le réglage automatique du niveau du sang contenu dans le réservoir comprend des moyens de réglage (25) qui sont conçus pour agir sur le degré de vide à l'intérieur dudit réservoir (2) sur la base d'une détection continue de la pression au niveau de la base du réservoir (2) et dudit degré de vide.

10. Réservoir selon la revendication 1,
**caractérisé en ce que** les moyens de fermeture pour fermer l'ouverture d'écoulement entre le réservoir (2) et le réservoir de cardiotomie (4) comprennent une colonne (11) qui peut coulisser lors d'un actionnement manuel et est munie d'un conduit (1 1 b) qui est ouvert à ces deux extrémités, respectivement, sur l'intérieur dudit réservoir de cardiotomie (4) et vers l'extérieur.
